# EUROPEAN PATENT APPLICATION

(11) **EP 1 734 039 A1**
(43) Date of publication of application: **20.12.2006**
(21) Application number: 05012616.8
(22) Date of filing: 13.06.2005
(51) Int. Cl.: C07D 295/12, C07C 275/40, C07D 249/12, C07D 285/08, C07D 295/22, C07D 251/52, C07D 495/04, C07D 471/08, A61K 31/17, A61K 31/53, A61P 25/28

(54) **Aryl urea compounds as BETA-secretase inhibitors**

(71) Applicant: Esbatech AG, 8952 Schlieren (CH)
(72) Inventor: Lüthi, Urs, 8330 Pfäffikon (CH); Danzhi, Huang, 8051 Zürich (CH); Kolb, Peter, 8057 Zürich (CH); Cecchini, Marco, 8057 Zürich (CH); Majeux, Nicolas, 8051 Zürich (CH); Dey, Fabian, 8055 Zürich (CH); Audétat, Stephan Valentin, 8107 Buchs (CH); Caflisch, Amedeo, 8057 Zürich (CH); Barberis, Alcide, 8057 Zürich (CH)
(74) Representative: Blum, Rudolf Emil

(57) **Abstract**

It has been found that compounds of formula I 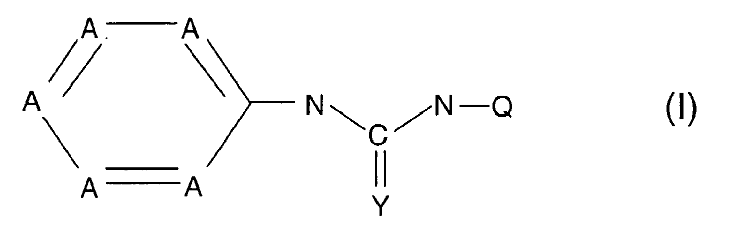 wherein
A is N or CR,
Y is O or S and
Q is an aromatic group or an araliphatic group having the following formula 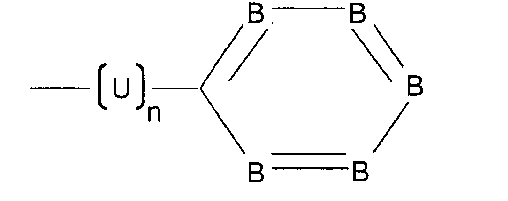 wherein
B = N or CR',
or Q has the following formula 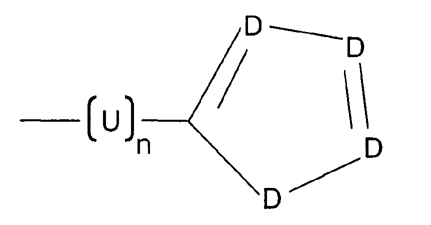 wherein
D = O or S or N or NR₄ or CR₅
are good β-secretase inhibitors for the treatment of Alzheimer's disease.

## Description

### Field of the Invention

This invention relates to aryl urea and aryl thiourea compounds, in particular to such compounds acting as beta-secretase inhibitors.

### Background Art

Alzheimer's disease (AD) is the most common form of dementia among older people, and affects parts of the brain that control thought, memory and language. Susceptibility to Alzheimer's disease increases with age, but Alzheimer's disease is not a normal part of the ageing process.

A characteristic of this disease is the presence of extracellular senile plaque, the major component of which is the β-amyloid peptide (Aβ). The hydrophobic, 39-43-amino-acid-long Aβ peptide is excised from the amyloid precursor protein (APP) by sequential cleavage by the so-called β- and γ-secretases.

Known genetic predispositions for AD mostly affect genes involved in Aβ generation or Aβ deposition. Since the Aβ peptide seems to play an important role in the pathogenesis of AD, current therapeutic strategies often focus on inhibition of Aβ deposition and generation. Inhibition of β-secretase activity represents an attractive option to achieve this goal.

Despite major efforts to identify novel β-secretase inhibitors by applying in vitro high-throughput screening (HTS) assays with purified soluble BACE-1 fragments and fluorogenic peptide substrates, the best progress towards efficient BACE-1 inhibition has been achieved so far by the use of peptidic transition-state mimetic compounds. However, for efficient inhibition of β-secretase in cells, their molecular weight must be reduced and their structure modified so as to allow for permeation of cellular membranes, the blood-brain barrier and for activity in the natural cellular environment.

There also exist some assays for identifying low molecular weight inhibitors of secretases that can block these membrane-bound enzymes at the natural location within intracellular compartments. Cell-based HTS assays, however, are generally faced with the problem that selection signals are often caused by compounds that interfere with cellular processes or pathways that are redundant with that of the target. For example, some compounds found by mammalian cell based assays impair the production of Aβ through the increase of the pH in intracellular compartments, or they function through protein phosphorylation, or they simply catalyze polymerization of Aβ, thus reducing the percentage of soluble peptide.

Some candidate compounds for inhibiting the production of Aβ peptide in a biological system have been proposed in US 5,814,646 and US 5,624,937.

Nevertheless, there is still a need for potent β-secretase inhibitors that directly inhibit β-secretase.

### Disclosure of the Invention

Hence, it is a general object of the invention to provide compounds that directly act as β-secretase inhibitors.

Now, in order to implement these and still further objects of the invention, which will become more readily apparent as the description proceeds, the β-secretase inhibitors of the present invention are manifested by the following formula I 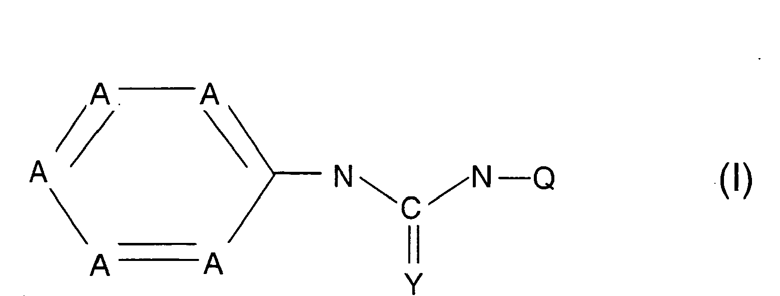 wherein
wherein
A = N or CR wherein
R is independently from each other selected from H, C₁-C₆-alkyl, C₁-C₆-alkoxy, halo-C₁-C₆-alkyl, e.g. CF₃, C₁-C₆-alkylcarbonyl, halogen, an -NR₂R₃ group
   wherein R2 and R3 are independently from each other H, linear or branched C1-C6-alkyl, in particular linear or branched C1-C4-alkyl, or R2 and R3 form together with the nitrogen to which they are bound an aliphatic or aromatic 5- or 6-membered one or more heteroatoms comprising heterocycle, e.g. a piperidino, a piperazino or a morpholino group, and wherein
Y = O or S,
Q = an aromatic group or an araliphatic group having the following formula 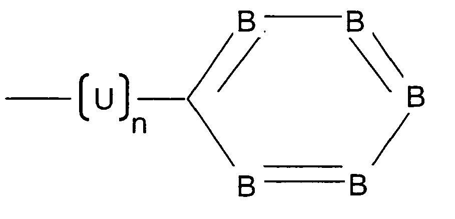
wherein
B = N or CR', wherein
R' is independently from each other selected from the group comprising hydrogen, halogen, in particular F, a C₁-C₆-alkyl group, in particular a C₁-C₄-alkyl group, an -NR₂R₃ group wherein R2 and R3 are independently from each other H, linear or branched Cl-C6-alkyl, in particular linear or branched C1-C4-alkyl, or R2 and R3 form together with the nitrogen to which they are bound an aliphatic or aromatic 5- or 6-membered one or more heteroatoms comprising heterocycle, e.g. a piperidino, a piperazino or a morpholino group, an amido group, an ester group, a 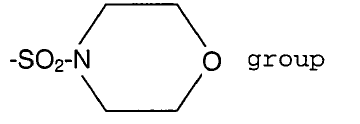 or two adjacent R' form a group 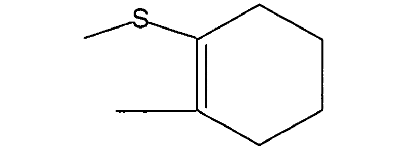
U = -CH₂-, C=O, -(CH₂)ₙS-, -(CH₂)ₙO-, -(CH₂)ₙNH-, or 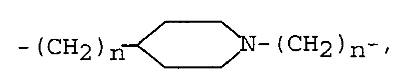 wherein the heterocycle is optionally substituted, in particular by one or two C₁-C₄-alkyl groups or such that a bicycle is formed, and
n independently from each other is 0, 1 or 2,
or Q has the following formula 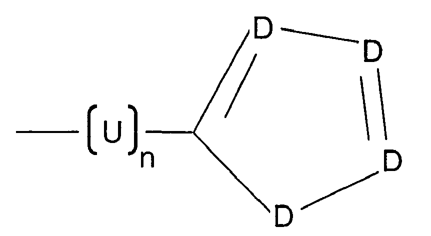
wherein
D = O or S or N or NR₄ or CR₅, wherein
R₄ is linear or branched C₁-C₆-alkyl, in particular C₁-C₄-alkyl
R₅ is independently from each other selected from C₁-C₆-alkylthio, aryl-C₁-C₆-alkylthio, aryloxy-C₁-C₆-alkyl, arylthio-C₁-C₆-alkyl, alkyloxy-C₁-C₆-alkyl, alkylthio-C₁-C₆-alkyl, C₁-C₆-alkyloxy, aryl-C₁-C₆-alkyloxy and optionally substituted linear or branched C₁-C₆-alkyl,
U and n are as defined above,
or pharmaceutically acceptable salts thereof,
with the proviso that in the case that n is 0 and

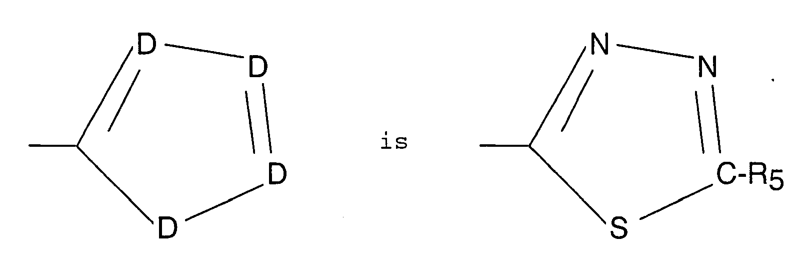

R₅ is substituted linear or branched C₁-C₆-alkyl, preferably linear or branched C₁-C₄-alkyl, with the substituent being selected from O-R₁₃ or S-R₁₃ or N-R₁₃R₁₃'with R₁₃ and R₁₃' being independently selected from the group consisting of unsubstituted or substituted 5- or 6-membered aryl, unsubstituted or substituted 5- or 6-membered heteroaryl,with the substituents of the aryl or heteroaryl group being as defined for R, linear or branched C₁-C₆-alkyl and C₅-C₆-cycloalkyl, in particular from the group consisting of O-R₁₃ or S-R₁₃ with R₁₃ being selected from the group consisting of 5- or 6-membered aryl, and linear or branched C₁-C₄-alkyl.

It has been found that compounds of formula (I) are efficient in inhibiting β-secretase activity. Thus, such compounds are suitable in the treatment and prophylaxis of β-secretase activity related diseases such as Alzheimer's disease, Down's syndrome, and advanced aging of brain.

In presently slightly preferred inhibitors
R is independently from each other selected from H, C₁-C₄-alkyl, C₁-C₄-alkoxy, halo-C₁-C₄-alkyl, C₁-C₄-alkylcarbonyl, halogen, an -NR₂R₃ group wherein R₂ and R₃ are independently from each other H or linear or branched C₁-C₄-alkyl, or R₂ and R₃ form together with the nitrogen to which they are bound an aliphatic or aromatic 5- or 6-membered one or more heteroatoms comprising heterocycle,
R' is independently from each other selected from the group consisting of hydrogen, halogen, in particular F, a C₁-C₄-alkyl group, an -NR₂R₃ group
wherein R₂ and R₃ are independently from each other H or linear or branched C₁-C₄-alkyl, or R₂ and R₃ form together with the nitrogen to which they are bound an aliphatic or aromatic 5- or 6-membered one or more heteroatoms comprising heterocycle, an amido group, an ester group, a 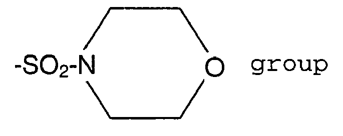 or two adjacent R' form a group 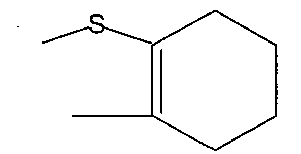 and
R₅ is independently from each other selected from C₁-C₄-alkylthio, aryl-C₁-C₄-alkylthio, aryloxy-C₁-C₄-alkyl, arylthio-C₁-C₄-alkyl, alkyloxy-C₁-C₄-alkyl, alkylthio-C₁-C₄-alkyl, C₁-C₄-alkyloxy, aryl-C₁-C₄-alkyloxy and optionally substituted linear or branched C₁-C₄-alkyl, preferably substituted linear or branched C₁-C₄-alkyl, with the substituent being selected from O-R₁₃ or S-R₁₃ with R₁₃ being selected from the group consisting of 5- or 6-membered aryl, 5- or 6-membered heteroaryl, linear or branched C₁-C₄-alkyl and C₅-C₆-cycloalkyl.

Preferred is a Q that is 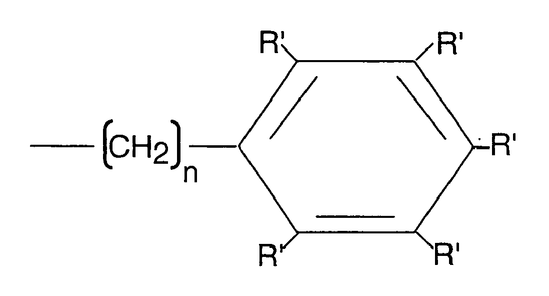 wherein R' and n are as defined above, and especially a Q selected from 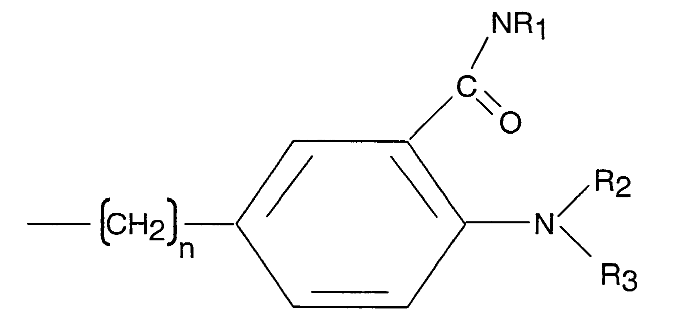 wherein
R₁ is optionally substituted C₁-C₄-alkyl,
   wherein the substituents are selected from optionally halogen substituted aryl, C₁-C₄-alkoxy, or morpholinyl,
R₂ = C₁-C₆-alkyl,
R₃ = C₁-C₆-alkyl,
or R₂ and R₃ form together with the nitrogen to which they are bound a 5-membered or a 6-membered aliphatic or aromatic ring, and
n = 0, 1 or 2, whereby in especially preferred embodiments
R₂ = C₁-C₄-alkyl, and
R₃ = C₁-C₄-alkyl,
or Q is 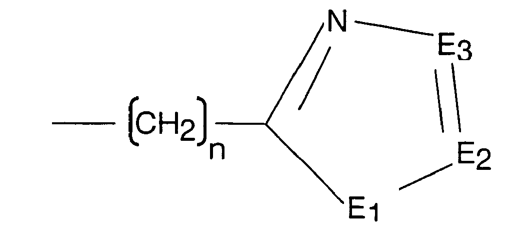
wherein
E₁ = NR₄ or S or O, wherein
R₄ is linear or branched C₁-C₄-alkyl,
E₂ = CR₅ or N, wherein
R₅ is C₁-C₄-alkylthio, aryl-C₁-C₄-alkylthio, aryloxy-C₁-C₄-alkyl,
E₃ = E₂ with the proviso that if E₂ is CR₅, E₃ is N and if E₂ is N, E₃ is CR₅, and
n is as defined above.

In a preferred embodiment
A = CR, wherein R is independently selected from H, CH₃, CH₂CH₃, OCH₃, COCH₃, Cl, Br, CF₃, more preferred selected from H, CH₃, CH₂CH₃, OCH₃, Cl, Br, CF_{3.}

In an also preferred embodiment, Q is 3-amido-4-amino-substituted phenyl a shown above with
R₁ = C₃-alkyl, C₁-C₄-alkoxy-C₂-C₃-alkyl, an optionally p-fluoro substituted phenyl-C₁-C₄-alkyl, and/or

R₂ = R₃ = -CH₃

or Q is 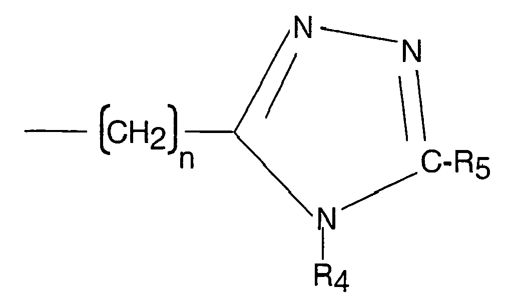 wherein
R₄ = linear or branched C₁-C₄-alkyl, in particular CH₃ or -CH(CH₃)₂
R₅ = C₁-C₄-alkylthio, and
n = 1 or 2,
or Q is 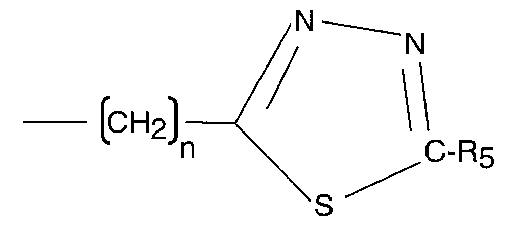
wherein
n = 0,
R₅ = aryloxy-C₁-C₄ alkyl, in particular phenoxy-C₁-C₄ alkyl, especially 1-phenoxy-ethyl,
or Q is 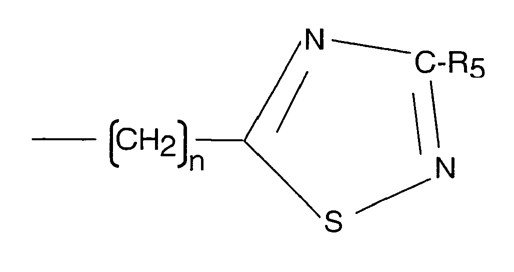
wherein
n = 0,
R₅ = C₁-C₄-alkylthio.

In a preferred embodiment, the compound is a compound of formula (II) 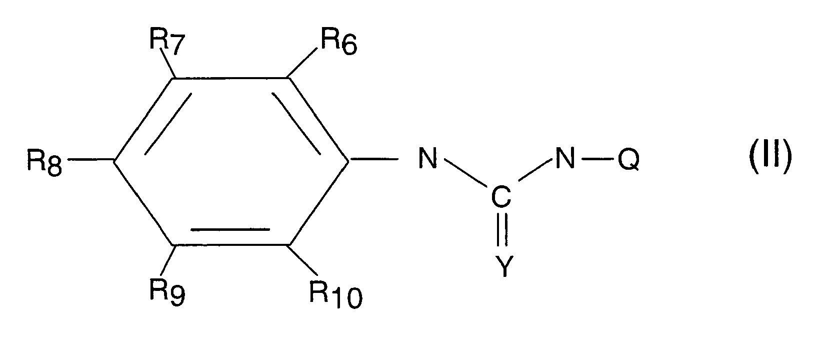 wherein
R₆ = H or halogen,
R₇ = H, C₁-C₄-alkyl, C₁-C₄-alkoxy, C₁-C₄-alkylcarbonyl, halo-C₁-C₄-alkyl or halogen,
R₈ = H, C₁-C₄-alkyl or halogen,
R₉ = H, C₁-C₄-alkyl, C₁-C₄-alkoxy or halo-C₁-C₄-alkyl,
R₁₀ = H or halogen, and
Y and Q are as defined above.

In more preferred compounds of formula (II)
R₆ = H or Br,
R₇ = H, C1, CH₃, OCH₃, CO-CH₃, CF₃,
R₈ = H, CH₂CH₃, CH₃, C1,
R₉ = H, CH₃, OCH₃, CF₃, and
R₁₀ = H, Br.

Also with regard to formula (II) Y and Q are as any Y and Q defined above with regard to formula (I) with the same preferences.

Especially preferred Q are 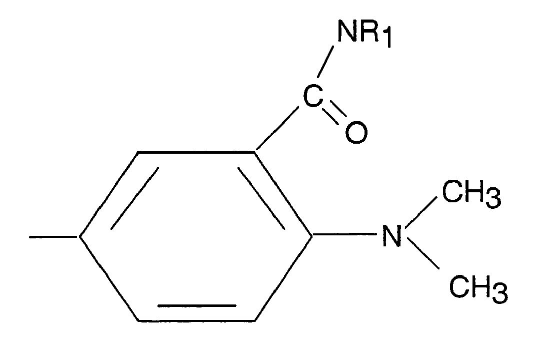 wherein
R₁ is optionally substituted C₁-C₄-alkyl,
   wherein the substituents are selected from optionally halogen substituted aryl, C₁-C₄-alkoxy, morpholinyl
R₂ = C₁-C₄-alkyl
R₃ = C₁-C₄-alkyl, and
n = 0 or 1
and preferably R₁ is CH₂CH₂CH₃, (CH₂)₃-OCH₃, (CH₂)₂-OCH₃, (CH₂)₃-OCH₂CH₃, CH₂CH₂-C₆C₅, p-F-benzyl, or 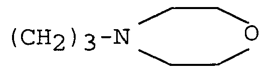 or Q is 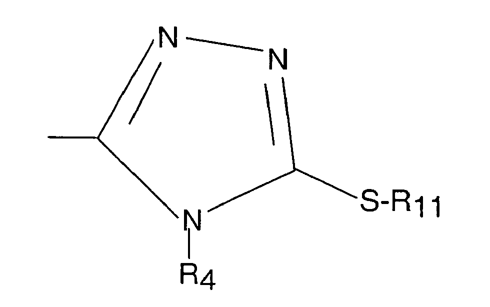 wherein R₄ is CH₃, CH(CH₃)₂, and R₁₁ is CH₃, CH₂CH₃, CH₂-C₆H₅,
or Q is 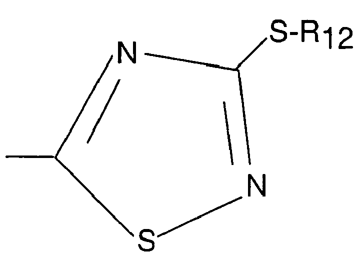 wherein R₁₂ is CH₃ or CH₂CH₃, in particular CH₂CH₃
or Q is 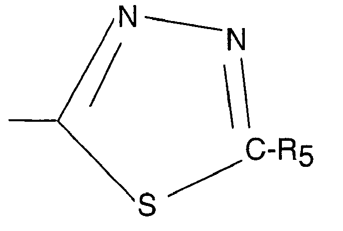 wherein R₅ is -(CH₂)ₘ(CR₁₃)(OAr),
wherein R₁₃ is H, C₁-C₄-alkyl, in particular methyl, Ar is phenyl or heteroaryl, in particular phenyl, and m = 0, 1 or 2.

As already mentioned above, the compounds of the present invention can be administered for prophylactic and/or therapeutic treatment of diseases related to the deposition of amyloid beta-protein, such as Alzheimer's disease, Down's syndrome, and advanced aging of the brain. In therapeutic applications, the compounds are administered to a host already suffering from the disease. The compounds will be administered in an amount sufficient to inhibit further deposition of senile plaques. The specific dose of compound(s) administered according to this invention to obtain therapeutic and/or prophylactic effects will, of course, be determined by the particular circumstances, such as the specific compound administered, the condition being treated, etc. A daily dose will contain a dosage level of from about 0.01 mg/kg to about 50 mg/kg of body weight of an active compound, preferably from about 0.05 mg/kg to about 20 mg/kg, for example from about 0.1 mg/kg to about 120 mg/kg.

The compound can be administered by a variety of routes including oral, rectal, transdermal, subcutaneous, intravenous, intramuscular and intranasal either as such, but preferable in a formulation comprising carriers adjuvants etc. Suitable pharmaceutically acceptable solid and liquid carriers and/or pharmaceutically acceptable adjuvants, such as stabilizing agents, emulsifyers, etc. are known in the art.

For example, a typical pharmaceutical composition for intramuscular injection would contain about one µg to one mg of the compound in from one to four milliliters of sterile buffered water. The typical pharmaceutical composition for intravenous infusion would contain about one to one hundred milligrams of the compound in from one hundred to five hundred milliliters of sterile Ringer's solution.

The pharmaceutical formulations are prepared by known procedures using known and readily available ingredients.

### Short Description of the Drawings

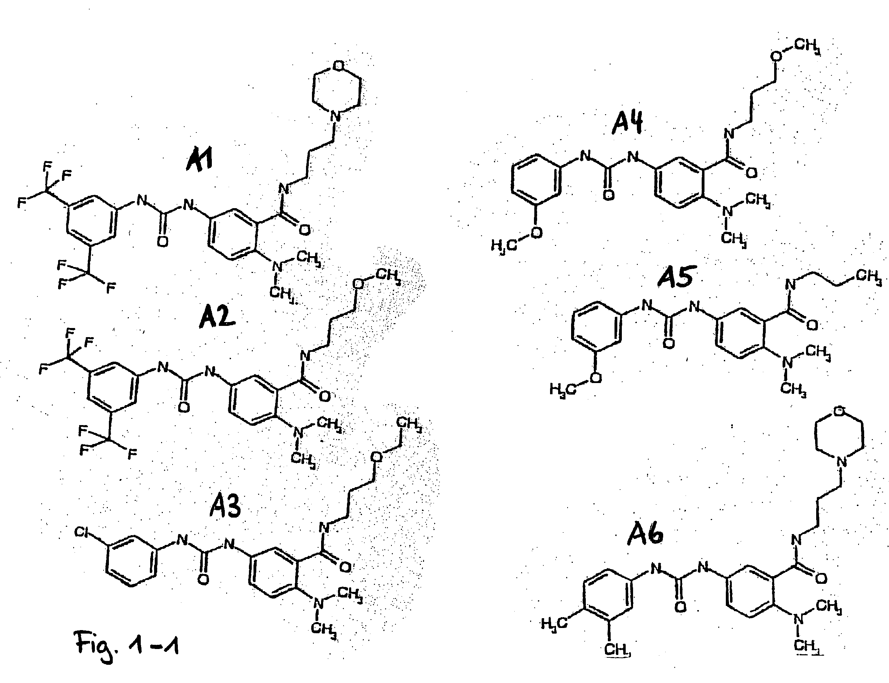

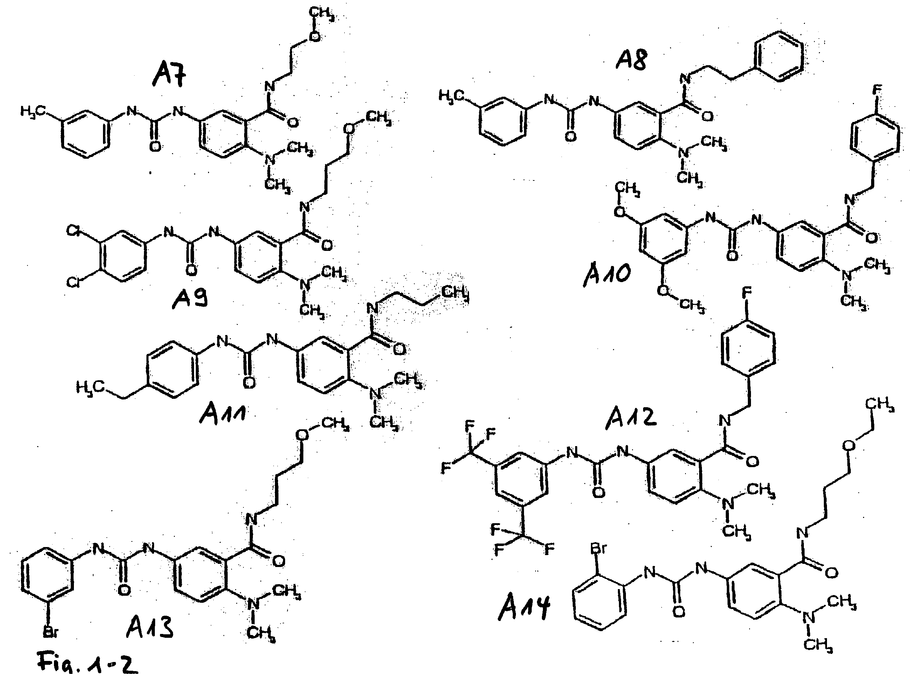

Figure 1 shows the structure formulas of compounds A1 to A14 of Tables 1 and 4

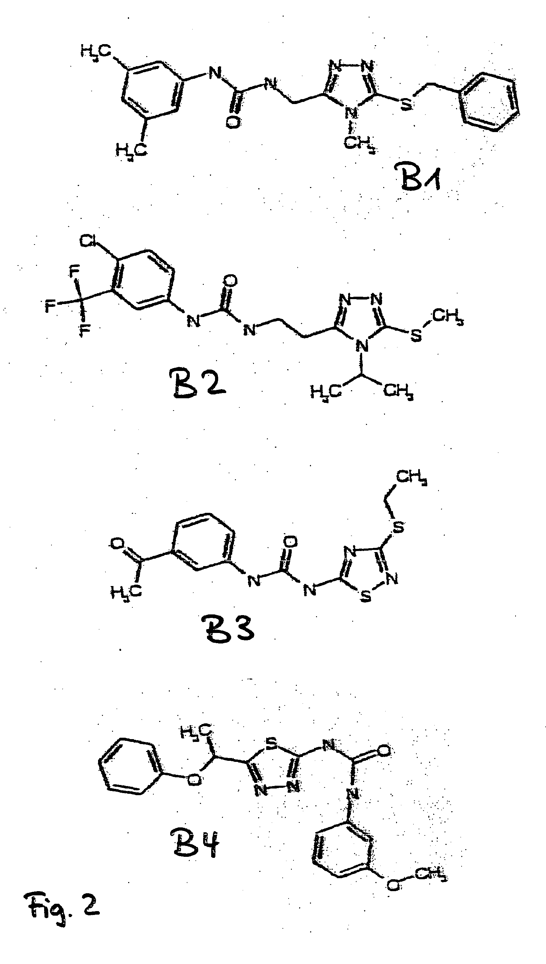

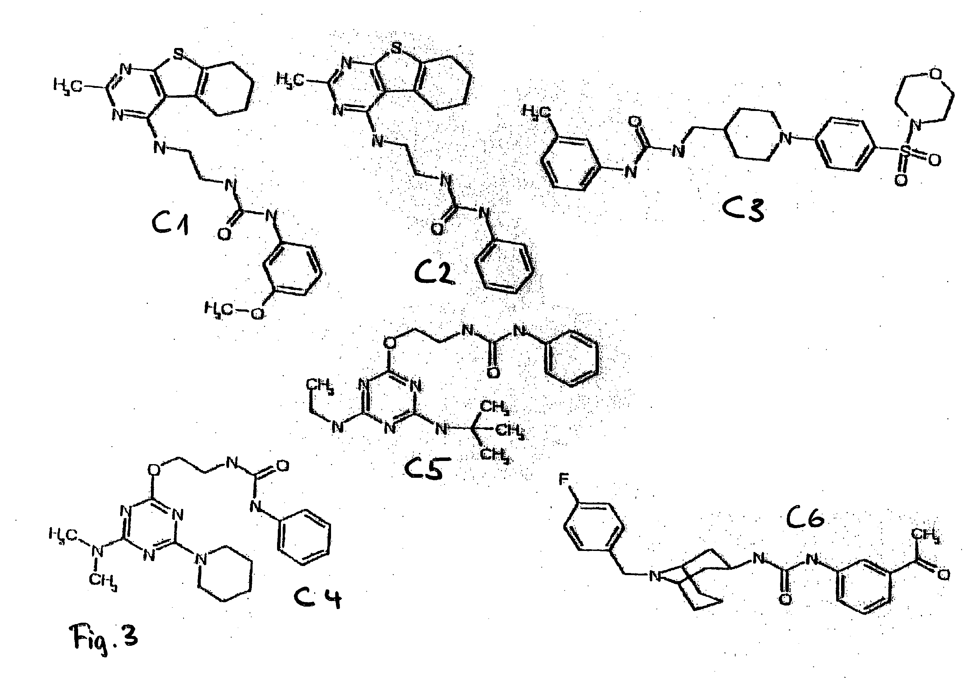

Figure 2 shows the structure formulas of compounds B1 to B4 of Tables 2 and 5
Figure 3 shows the structure formula of compounds C1 to C6 of Tables 3 and 6.

### Modes for Carrying Out the Invention

Specific compounds and their β-secretase inhibiting effects are further described below.

Tables 1 to 3 make a relation between compound designation and structure.

**Table 1:**

| | | | | | |
|---|---|---|---|---|---|
| 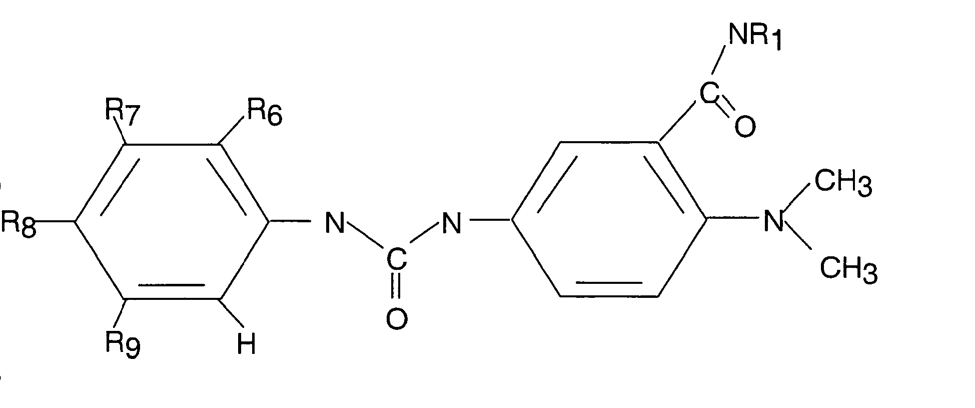 | | | | | |

| **Compound** | **R₁** | **R₆** | **R₇** | **R₈** | **R₉** |
|---|---|---|---|---|---|
| A1 | 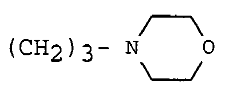 | H | CF₃ | H | CF₃ |
| A2 | (CH₂)₃-OCH₃ | H | CF₃ | H | CF₃ |
| A3 | (CH₂)₃-OCH₂CH₃ | H | Cl | H | H |
| A4 | (CH₂)₃-OCH₃ | H | H | H | OCH₃ |
| A5 | (CH₂)₂-CH₃ | H | H | H | OCH₃ |
| A6 | 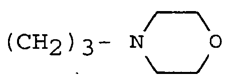 | H | H | CH₃ | CH₃ |
| A7 | (CH₂)₂-OCH₃ | H | CH₃ | H | H |
| A8 | (CH₂)₂-C₆H₅ | H | CH₃ | H | H |
| A9 | (CH₂)₃-OCH₃ | H | Cl | Cl | H |
| A10 | CH₂-p-F-C₆H₄ | H | OCH₃ | H | OCH₃ |
| A11 | (CH₂)₂-CH₃ | H | H | CH₂CH₃ | H |
| A12 | CH₂-p-F-C₆H₄ | H | CF₃ | H | CF₃ |
| A13 | (CH₂)₃-OCH₃ | H | H | H | Br |
| A14 | (CH₂)₃OCH₂CH₃ | Br | H | H | H |

**Table 2:**

| | | | | | | | | | |
|---|---|---|---|---|---|---|---|---|---|
| 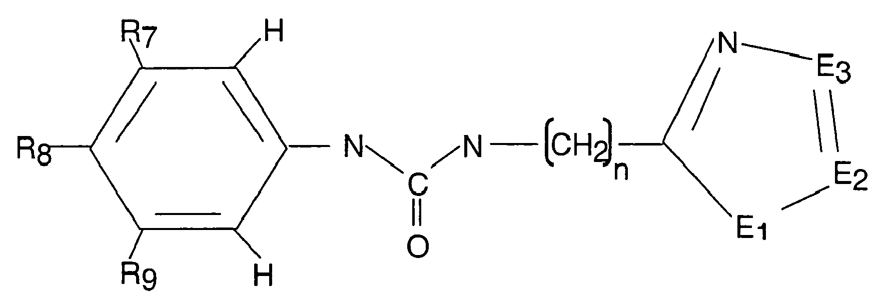 | | | | | | | | | |

| **Compo und** | **R₇** | **R₈** | **R₉** | **E₁** | **R₄** | **E₂** | **E₃** | **R₅** | **n** |
|---|---|---|---|---|---|---|---|---|---|
| B1 | CH₃ | H | CH₃ | NR₄ | CH₃ | CR₅ | N | S-CH₂-C₆H₅ | 1 |
| B2 | CF₃ | Cl | H | NR₄ | CH(CH3)2 | CR₅ | N | S-CH₃ | 2 |
| B3 | H | H | COCH₃ | S | - | N | CR₅ | S-CH₂CH₃ | 0 |
| B4 | OCH₃ | H | H | S | - | CR₅ | N | C(CH₃)(O-C₆H₅) | 0 |

**Table 3:**

| 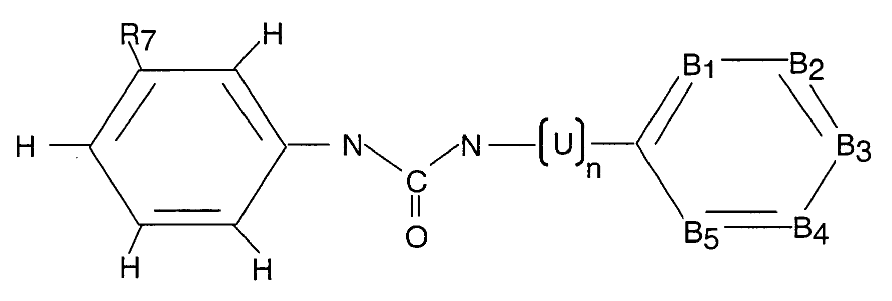 | | | | | | | |
|---|---|---|---|---|---|---|---|
| **Compo und** | **R₇** | **-[U]ₙ-** | **B₁** | **B₂** | **B₃** | **B₄** | **B₅** |
| C1 | OCH₃ | -(CH₂)₂NH- | N | CCH₃ | N | 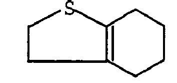 | |
| C2 | H | -(CH₂)₂NH- | N | CCH₃ | N | 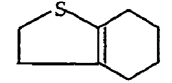 | |
| C3 | CH₃ | 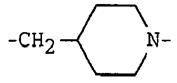 | CH | CH | 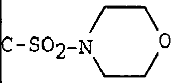 | CH | CH |
| C4 | H | -(CH₂)₂O- | N | 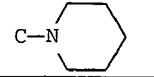 | N | C-N(CH₃)₂ | N |
| C5 | H | -(CH₂)₂O- | N | CNH(CH₂CH₃) | N | C-NHC(CH₃)₃ | N |
| C6 | COCH₃ | 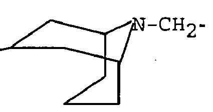 | CH | CH | CF | CH | CH |

These compounds have been tested for their performance as β-secretase inhibitors.

The results of three different tests performed are listed in Tables 4 to 6 below.

The tests performed were
a) Aβ1-40 (Sw) bioassay, which measures the amount of the amyoid peptide Aβ1-40 in the supernatant of Swedish APP695 transgenic HEK293 cells in the presence of the various BACE inhibitors via ELISA (enzyme-linked immunosorbent assay). In the table, the inhibitory concentration that reduces Aβ1-40 secretion to 50 % is indicated (IC50), or the % reduction of Aβ1-40 secretion at the indicated concentration.
b) SEAP bioassay, which measures the amount of the secreted reporter enzyme SEAP (secreted alkaline phosphatase) in the supernatant of transiently transfected HEK293 cells. A SEAP-APP(Sw)695 fusion protein is transiently expressed in HEK293 cells in the presence of the various BACE inhibitors. Secretion of the SEAP moiety upon cleavage at the APP β-site is quantitated via a luminescence readout. In the table, the inhibitory concentration that reduces secreted SEAP activity to 50 % is indicated (IC50), or the % reduction of secreted SEAP activity at the indicated concentration.
c) FRET assay, which measures the activity of recombinant BACE enzyme in the presence of the various BACE inhibitors via a FRET (fluorescence resonance energy transfer)-based readout. In the table, the inhibitory concentration that reduces the activity of BACE to 50 % is indicated (IC50), or the % reduction of the activity of BACE at the indicated concentration.
d) An additional in silico test was performed for the compounds listed in Tables 1 to 3. The compounds were docked with the FFLD approach (Budin et al., Biol. Chem. 382, 1365-1372, 2001) and their binding energy was evaluated with the LIECE method (Huang and Caflisch, J. Med. Chem. 47, 5791-5797, 2004). The affinity evaluated with LIECE is in the low micromolar range for most of these compounds.

**Table 4**

| **Compo und** | **Aβ1-40 (Sw) bioassay (cell-based)** | **SEAP bioassay (cell-based)** | **FRET assay (in vitro)** | **LIECE Ki[µM]** |
|---|---|---|---|---|
| A1 | IC50 3.0 µM | IC50 3.5 µM | IC50 58 µM | 8.11 |
| A2 | IC50 3.2 µM | 27 (3 µM) | IC50 284 µM | 9.35 |
| A3 | IC50 2.6 µM | 23 (3 µM) | IC50 97 µM | 9.81 |
| A4 | IC50 7.5 µM | 21 (6 µM) | 33 (100 µM) | 10.26 |
| A5 | IC50 14.3 µM | 0 (6 µM) | 16 (100 µM) | 15.99 |
| A6 | IC50 23 µM | 19 (12.5 µM) | 21 (100 µM) | 17.56 |
| A7 | IC50 12.9 µM | 0 (12.5 µM) | 0 (100 µM) | 18.64 |
| A8 | IC50 5.6 µM | 14 (3 µM) | 35 (100 µM) | 32.34 |
| A9 | IC50 5.9 µM | 17 (3 µM) | IC50 46 µM | 32.56 |
| A10 | IC50 3.1 µM | 10 (1.6 µM) | IC50 64 µM | 34.71 |
| A11 | IC50 5.2 µM | 0 (6 µM) | 20 (200 µM) | 38.37 |
| A12 | IC50 4.2 µM | 14 (1.6 µM) | IC50 131 µM | 39.41 |
| A13 | IC50 3.8 µM | 25 (3 µM) | 37 (100 µM) | 50.09 |
| A14 | IC50 7.8 µM | 0 (6 µM) | 0 (100 µM) | 66.41 |

**Table 5:**

| **Compo und** | **Aβ1-40 (Sw) bioassay** (cell-based) | **SEAP bioassay (cell-based)** | **FRET assay (in vitro)** | **LIECE Ki[µM]** |
|---|---|---|---|---|
| B1 | IC50 18 µM | 0 (50 µM) | 0 (500 µM) | 11.85 |
| B2 | IC50 40 µM | 0 (12.5 µM) | 0 (250 µM) | 28.53 |
| B3 | IC50 1.6 µM | IC50 10 µM | 0 (25 µM) | 29.60 |
| B4 | IC50 13 µM | 0 (12.5 µM) | 0 (500 µM) | 34.97 |

**Table 6:**

| **Compo und** | **Aβ1-40 (Sw) bioassay (cell-based)** | **SEAP bioassay (cell-based)** | **FRET assay (in vitro)** | **LIECE Ki[µM]** |
|---|---|---|---|---|
| C1 | 21 (6 µM) | 0 (12.5 µM) | 0 (500 µM) | 15.37 |
| C2 | IC50 13 µM | 0 (12.5 µM) | 0 (500 µM) | 33.69 |
| C3 | IC50 22 µM | 0 (12.5 µM) | 0 (50 µM) | 15.96 |
| C4 | 31 (12.5 µM) | 0 (12.5 µM) | 0 (500 µM) | 23.49 |
| C5 | IC50 10-20 µM | 0 (25 µM) | 0 (500 µM) | 32.95 |
| C6 | 22 (25 µM) | IC50 35 µM | IC50 490 µM | 48.10 |

While there are shown and described presently preferred embodiments of the invention, it is to be distinctly understood that the invention is not limited thereto but may be otherwise variously embodied and practiced within the scope of the following claims.

## Claims

1. β-secretase inhibitors of formula I 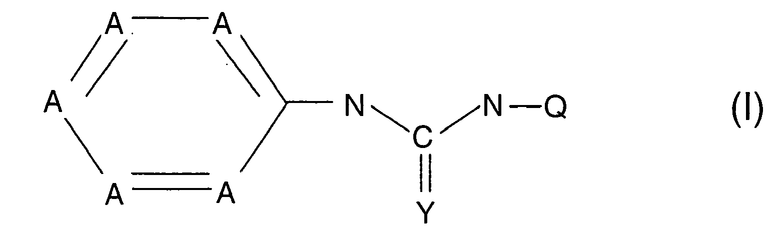 wherein
A = N or CR wherein
R is independently from each other selected from H, C₁-C₆-alkyl, C₁-C₆-alkoxy, halo-C₁-C₆-alkyl, C₁-C₆-alkylcarbonyl, halogen, an -NR₂R₃ group wherein R2 and R3 are independently from each other H, linear or branched C1-C6-alkyl, in particular linear or branched Cl-C4-alkyl, or R2 and R3 form together with the nitrogen to which they are bound an aliphatic or aromatic 5- or 6-membered one or more heteroatoms comprising heterocycle, and wherein
Y = O or S
Q = an aromatic group or an araliphatic group having the following formula 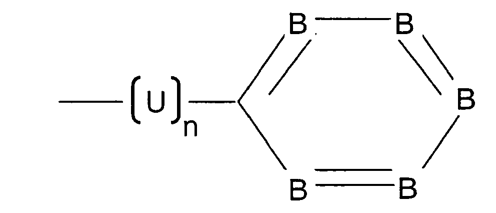
wherein
B = N or CR', wherein
R' is independently from each other selected from the group comprising hydrogen, halogen, in particular F, a C₁-C₆-alkyl group, in particular a C₁-C₄-alkyl group, an -NR₂R₃ group wherein R2 and R3 are independently from each other H, linear or branched Cl-C6-alkyl, in particular linear or branched C1-C4-alkyl, or R2 and R3 form together with the nitrogen to which they are bound an aliphatic or aromatic 5- or 6-membered one or more heteroatoms comprising heterocycle, an amido group, an ester group, a 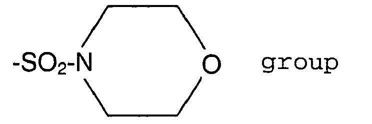 or two adjacent R' form the group 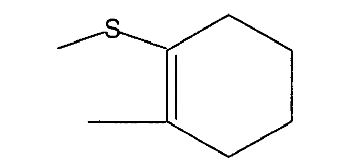
-U- = -CH₂-, C=O, -(CH₂)ₙS-, -(CH₂)ₙO-, -(CH₂)ₙNH-, or 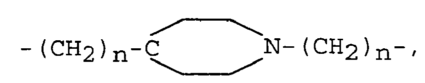 wherein the heterocycle is optionally substituted, in particular by one or two C₁-C₄-alkyl groups, or such that a bicycle is formed, and
n independently from each other is 0, 1 or 2
or Q has the following formula 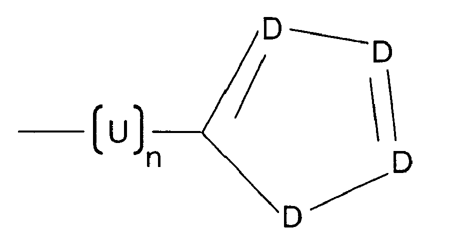
wherein
D = O or S or N or NR₄ or CR₅, wherein
R₄ is linear or branched C₁-C₆-alkyl, in particular C₁-C₄-alkyl
R₅ is independently from each other selected from C₁-C₆-alkylthio, aryl-C₁-C₆-alkylthio, aryloxy-C₁-C₆-alkyl, arylthio-C₁-C₆-alkyl, alkyloxy-C₁-C₆-alkyl, alkylthio-C₁-C₆-alkyl, C₁-C₆-alkyloxy, aryl-C₁-C₆-alkyloxy and optionally substituted linear or branched C₁-C₆-alkyl,
U and n are as defined above,
or pharmaceutically acceptable salts thereof
as pharmaceutical, with the proviso that in the case that n is 0 and 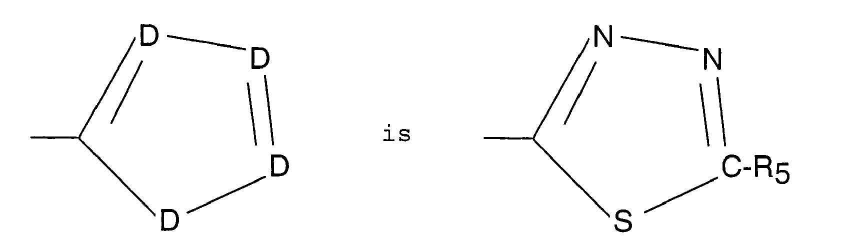
R₅ is substituted linear or branched C₁-C₆-alkyl, preferably linear or branched C₁-C₄-alkyl, with the substituent being selected from O-R₁₃ or S-R₁₃ or N-R₁₃R_{I3}'with R₁₃ and R₁₃' being independently selected from the group consisting of unsubstituted or substituted 5- or 6-membered aryl, unsubstituted or substituted 5- or 6-membered heteroaryl, with the substituents of the aryl or heteroaryl group being as defined for R, linear or branched C₁-C₆-alkyl and C₅-C₆-cycloalkyl, in particular from the group consisting of O-R₁₃ or S-R₁₃ with R₁₃ being selected from the group consisting of 5- or 6-membered aryl, and linear or branched C₁-C₄-alkyl.

2. The β-secretase inhibitors of claim 1,
wherein
R is independently from each other selected from H, C₁-C₄-alkyl, C₁-C₄-alkoxy, halo-C₁-C₄-alkyl, C₁-C₄-alkylcarbonyl, halogen, an -NR₂R₃ group wherein R₂ and R₃ are independently from each other H or linear or branched C₁-C₄-alkyl, or R₂ and R₃ form together with the nitrogen to which they are bound an aliphatic or aromatic 5- or 6-membered one or more heteroatoms comprising heterocycle,
R' is independently from each other selected from the group consisting of hydrogen, halogen, in particular F, a C₁-C₄-alkyl group, an -NR₂R₃ group
wherein R₂ and R₃ are independently from each other H or linear or branched C₁-C₄-alkyl, or R₂ and R₃ form together with the nitrogen to which they are bound an aliphatic or aromatic 5- or 6-membered one or more heteroatoms comprising heterocycle, an amido group, and an ester group, a 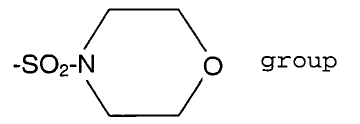 or two adjacent R' form a group 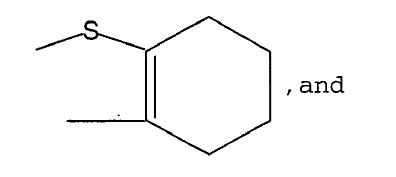
R₅ is independently from each other selected from C₁-C₄-alkylthio, aryl-C₁-C₄-alkylthio, aryloxy-C₁-C₄-alkyl, arylthio-C₁-C₄-alkyl, alkyloxy-C₁-C₄-alkyl, alkylthio-C₁-C₄-alkyl, C₁-C₄-alkyloxy, aryl-C₁-C₄-alkyloxy and optionally substituted linear or branched C₁-C₄-alkyl, preferably substituted linear or branched C₁-C₄-alkyl, with the substituent being selected from OR₁₃ or S-R₁₃ with R₁₃ being selected from the group consisting of 5- or 6-membered aryl, 5- or 6-membered heteroaryl, linear or branched C₁-C₄-alkyl and C₅-C₆-cycloalkyl.

3. The β-secretase inhibitors of claim 1 or 2
wherein Q is an aromatic or araliphatic group as defined above or
Q is 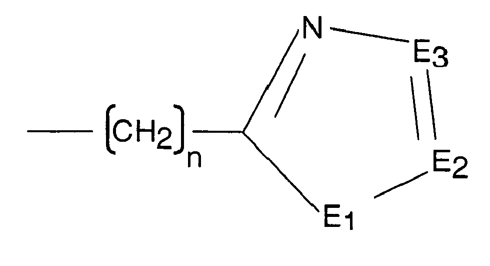
wherein
E₁ = NR₄ or S or O, wherein
R₄ is linear or branched C₁-C₄-alkyl,
E₂ = CR₅ or N, wherein
R₅ is C₁-C₄-alkylthio, aryl-C₁-C₄-alkylthio, aryloxy-C₁-C₄-alkyl,
E₃ = E₂ with the proviso that if E₂ is CR₅, E₃ is N and if E₂ is N, E₃ is CR₅, and
n is as defined above.

4. The β-secretase inhibitors of anyone of the preceding claims with formula II 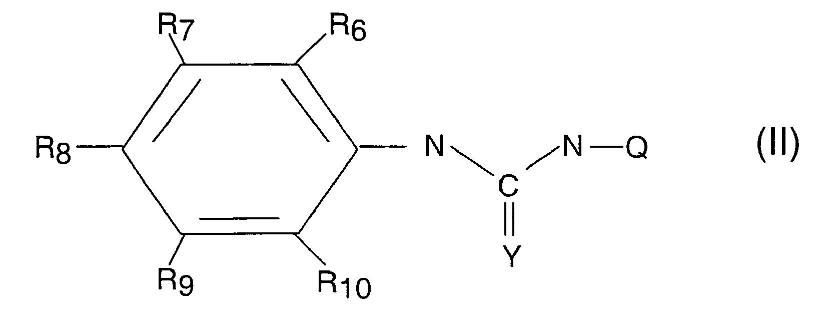 wherein
R₆ = H or halogen
R₇ = H, C₁-C₄-alkyl, C₁-C₄-alkoxy, C₁-C₄-alkylcarbonyl, halo-C₁-C₄-alkyl or halogen,
R₈ = H, C₁-C₄-alkyl or halogen,
R₉ = H, C₁-C₄-alkyl, C₁-C₄-alkoxy or halo-C₁-C₄-alkyl,
R₁₀ = H or halogen
Y and Q are as defined above, and
wherein Q is a group of the following formula 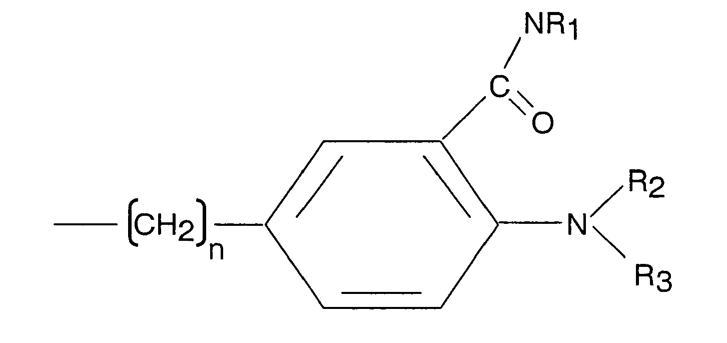
wherein
R₁ is optionally substituted C₁-C₄-alkyl,
wherein the substituents are selected from optionally halogen substituted aryl, C₁-C₄-alkoxy, morpholinyl,
R₂ = C₁-C₆-alkyl,
R₃ = C₁-C₆-alkyl, or
R2 and R3 form together with the nitrogen to which they are bound a 5-membered or a 6-membered aromatic or aliphatic heterocycle, and
n = 0, 1 or 2,
or Q is a group of the following formula 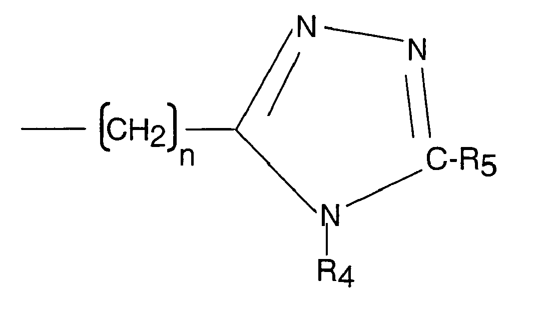 wherein
R₄ = linear or branched C₁-C₄-alkyl, in particular CH₃ or CH(CH₃)₂,
R₅ = is C₁-C₄-alkylthio or aryl-C₁-C₄-alkylthio, and
n = 1 or 2,
or Q is a group of the following formula 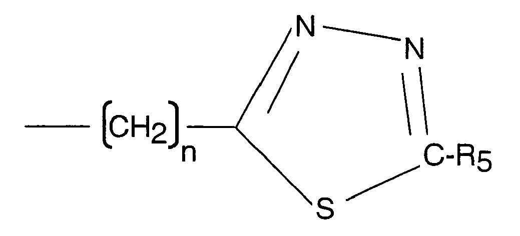 wherein
n = 0,
R₅ = aryloxy-C₁-C₄ alkyl, in particular phenoxy-C₁-C₄ alkyl, especially 1-phenoxy-ethyl,
or Q is a group of the following formula 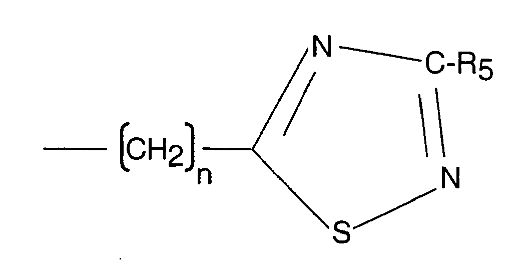 wherein
n = 0,
R₅ = C₁-C₄ alkylthio,
or pharmaceutically acceptable salts thereof.

5. The β-secretase inhibitor of claim 4
wherein
R₆ = H or Br,
R₇ = H, Cl, CH₃, OCH₃, CO-CH₃, CF₃,
R₈ = H, CH₂CH₃, CH₃, Cl,
R₉ = H, CH₃, OCH₃, CF₃,
R₁₀ = H, Br,
or pharmaceutically acceptable salts thereof.

6. The β-secretase inhibitor of claim 4 or 5
wherein Q is a group of the following formula 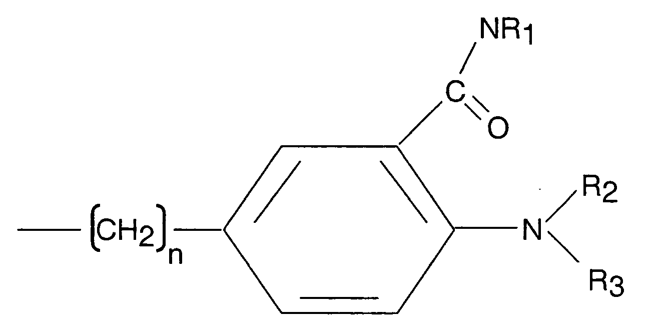 wherein
R₁ is optionally substituted C₁-C₄-alkyl,
wherein the substituents are selected from optionally halogen substituted aryl, C₁-C₄-alkoxy, morpholinyl,
R₂ = C₁-C₄-alkyl,
R₃ = C₁-C₄-alkyl, and
n = 0 or 1.

7. The β-secretase inhibitor of claim 6,
wherein Q is 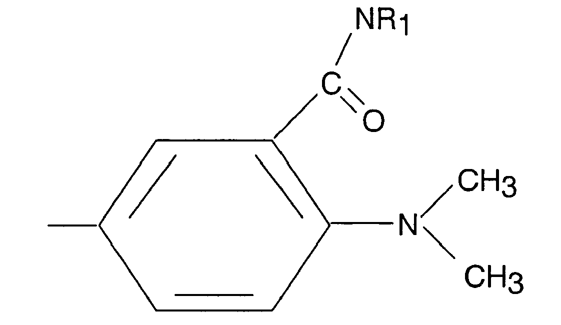 or pharmaceutically acceptable salts thereof.

8. The β-secretase inhibitor of claim 7,
wherein R₁ is CH₂CH₂CH₃, (CH₂)₃-OCH₃, (CH₂)₂-OCH₃, (CH₂)₃-OCH₂CH₃, CH₂CH₂-C₆C₅, CH₂-p-F-C₆H₄, or

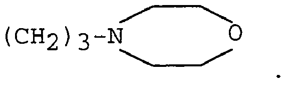

9. The β-secretase inhibitor of claim 4 or 5,
wherein Q is 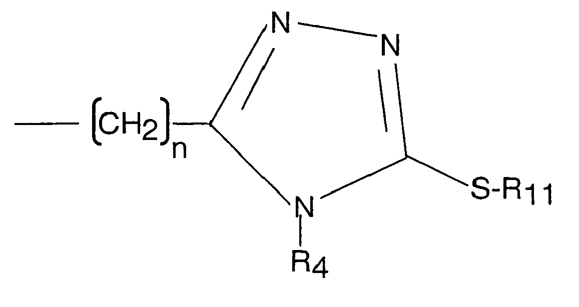 and wherein
n is 1 or 2,
R₄ is CH₃, CH(CH₃)₂, and
R₁₁ is CH₃, CH₂CH₃, CH₂-C₆H₅,
or pharmaceutically acceptable salts thereof.

10. The β-secretase inhibitor of claim 4 or 5, wherein Q is 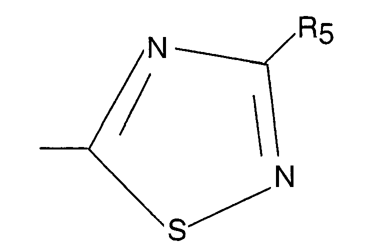 and wherein R₅ is as defined above,
or pharmaceutically acceptable salts thereof.

11. The β-secretase inhibitor of claim 10,
wherein Q is 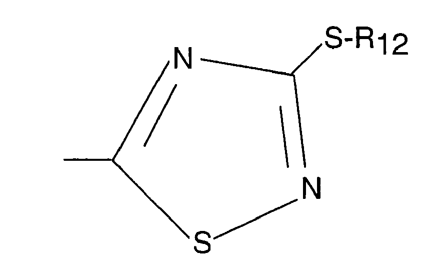 wherein R₁₂ is CH₃ or CH₂CH₃, in particular CH₂CH₃,
or pharmaceutically acceptable salts thereof.

12. The β-secretase inhibitor of claim 4 or 5, wherein Q is 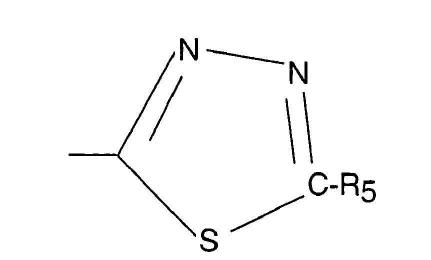 and wherein R₅ is as defined above,
or pharmaceutically acceptable salts thereof.

13. The β-secretase inhibitor of claim 12
wherein R₅ is (CH₂)ₘ-(CR₁₃) (OAr)
wherein R₁₃ is H, C₁-C₄-alkyl, in particular methyl, Ar is phenyl or heteroaryl, in particular phenyl, and m = 0, 1 or 2, or pharmaceutically acceptable salts thereof.

14. Use of a β-secretase inhibitor of anyone of the preceding claims, or a pharmaceutically acceptable salt thereof, in the manufacture of a medicament for inhibiting the production and/or the accumulation of amyloid beta-protein in warm blooded mammals, in particular human beings, especially for the treatment of Alzheimer's disease and or Down's syndrome and/or aging of brain.

15. A pharmaceutical composition comprising at least one β-secretase inhibitor of anyone of claims 1 to 13, or a pharmaceutically acceptable salt thereof, together with a pharmaceutically acceptable carrier and optionally one or more adjuvants.
